# EUROPEAN PATENT SPECIFICATION

(11) **EP 3 512 448 B1**
(45) Date of publication and mention of the grant of the patent: **31.03.2021**
(21) Application number: 17777713.3
(22) Date of filing: 15.09.2017
(51) Int. Cl.: A61B 18/20, A61B 18/00, A61B 17/00

(54) **DUAL-WAVELENGTH LASER TREATMENT**
LASERBEHANDLUNG MIT ZWEI WELLENLÄNGEN
TRAITEMENT LASER À DOUBLE LONGUEUR D'ONDE

(30) Priority: 16.09.2016 US 201662396003 P
(43) Date of publication of application: 24.07.2019
(73) Proprietor: Boston Scientific Scimed, Inc., Maple Grove, MN 55311-1566 (US)
(72) Inventor: XUAN, Rongwei, Jason, Fremont, CA 94538 (US); KOULLICK, Edouard, A., Golden Valley, MN 55416 (US); HASENBERG, Thomas, Campbell, CA 95008 (US); HWANG, Jieun, Hwasun-gun Jeollanam-do 58126 (KR); KIM, Hyejin, Changwon-si Gyeongsangnam-do 51447 (KR); PYO, Hanjae, Busanjingu Susan 47352 (KR); KANG, Hyun Wook, Busan (KR); GONG, Jason, San Jose, CA 95129 (US); YANG, Xirong, Fremont, CA 95129 (US); YU, Honggang, San Jose, CA 95129 (US)
(74) Representative: Pfenning, Meinig & Partner mbB
(86) International application number: PCT/US2017/051741
(87) International publication number: WO 2018/053241

(56) References cited:
- WO-A1-01/08579
- WO-A1-01/74265
- US-A1- 2003 135 205
- US-A1- 2007 129 712
- US-A1- 2007 219 601
- US-A1- 2010 179 523

## Description

### Technical Field

Aspects of the present disclosure generally relate to methods and apparatuses for dual-wavelength laser treatment. In particular, aspects of this disclosure relate to dual-wavelength photocoagulation.

### Background

Laser energy has a variety of beneficial uses, many of which are wavelength specific. Some laser systems include a plurality of laser sources for this purpose, such as a first laser source configured to discharge a first laser energy at a first wavelength, and a second laser source configured to discharge a second laser energy at a second wavelength. For example, the first wavelength may be such that the first laser energy is strongly absorbed by hemoglobin in blood, making the first laser energy very effective in cutting and vaporizing vascular tissue; and the second wavelength may be such that the second laser energy is not strongly absorbed by hemoglobin, instead penetrating deeply into the tissue, causing heat sufficient to coagulate blood and/or stop bleeding.

As a further example, document WO 01/74265 A1 discloses a method for treating vascular disorders includes irradiating an abnormal blood vessel with two pulses of electromagnetic radiation. One of the pulses has a longer wavelength than the other. The longerwavelength pulse is delivered concurrently with or at a relatively short time interval after the shorter wavelength pulse. Neither pulse delivers sufficient fluence to permanently damage the vessel if used alone. When the pulses are used in combination, the vessel is transformed by the shorter wavelength pulse to a condition where coagulation of blood in the vessel and permanent damage to the vessel can be caused by the second pulse.

Photocoagulation is a therapeutic method known to achieve hemostasis during laser treatment on benign prostate hyperplasia (BPH). Therefore, in some cases, the first laser energy is discharged to vaporize tissue until a bleeding occurs, after which the second laser energy is discharged to stop the bleed. In this example, the coagulative effectiveness of the medical laser system is determined exclusively by characteristics of the second laser energy, such as power level and/or pulse timing, meaning that the first laser energy plays no role in coagulation. Applying a single laser wavelength laser (i.e., only the second laser energy) often involves lengthy irradiation, insufficient tissue coagulation, or even incomplete hemostasis.

Further improvement is required to address these issues.

### SUMMARY

The invention is defined in claim 1. A laser system in accordance with the invention comprises: a first laser source configured to output a first laser energy at a first wavelength; a second laser source configured to output a second laser energy at a second wavelength; and a combiner configured to receive the first and second laser energies and output a dual-wavelength laser energy, wherein the first and second wavelengths are different, and to output said first and second laser energies simultaneously. The laser system further includes a controller configured to receive user input from an input device and to place the laser system in a vaporization mode to output the first laser energy onto a targeted location. The controller is further configured to place the laser system in a dual-wavelength coagulation mode to output the dual-wavelength laser energy onto the targeted location, responsive to said user input.

The first wavelength occurs within a vaporization wavelength range of 400nm-600nm, and the second wavelength occurs within a coagulation wavelength range of 780nm-3.0µm. For example, the first wavelength may be 532nm, and the second wavelength may be 980nm. The first laser energy may be output from the first laser source at a first power level, and the second laser energy may be output from the second laser source at a second power level. The first and second power levels may be identical or different. For example, the first power level may be 20W, and the second power level may be 40W. The first laser energy may be output as a pulsed waveform, and the second laser energy may be output as a continuous waveform.

Another aspect of this disclosure not forming part of the invention as claimed is a method. The method may comprise: powering a laser system including a first laser source configured to output a first laser energy at a first wavelength, a second laser source configured to output a second laser energy at a second wavelength, and a combiner configured to receive the first and second laser energies and output a dual-wavelength laser energy, wherein the first and second wavelengths are different, and first and second laser energies are output simultaneously; placing the laser system in a vaporization mode; outputting the first laser energy onto a targeted location; placing the laser system in a coagulation mode; and outputting the dual-wavelength laser energy onto the targeted location.

According to this aspect, the method may further comprise performing a vaporization treatment on a tissue at the targeted location with the first laser energy, and/or coagulating a tissue at the targeted location with the second laser energy. The first wavelength may be selected from a vaporization wavelength range of 400nm-600nm, and the second wavelength may be selected from a coagulation range of 780nm-3.0µm. The first laser energy may be output from the first laser source at a first power level, the second laser energy may be output from the second laser source at a second power level. The first and second power levels may be identical or different. For example, the first power level may be 20W, and the second power level may be 40W. The first laser energy may be output as a pulsed waveform, and the second laser energy may be output as a continuous waveform.

It may be understood that both the foregoing summary and the following detailed descriptions are exemplary and explanatory only, neither being restrictive of the inventions claimed below.

### BRIEF DESCRIPTION OF THE DRAWINGS

The accompanying drawings are incorporated in and constitute a part of this specification. These drawings illustrate aspects of the present disclosure that, together with the written descriptions, serve to explain the principles of this disclosure.
FIG. 1 is a schematic diagram of an exemplary surgical laser system, according to aspects of this disclosure.
FIGs. 2A through 2E depict the coagulative effects of laser energy output, according to aspects of this disclosure.
FIG. 3 depicts a comparison of the effects depicted in FIGs. 2A-E.
FIG. 4 is a flow chart depicting the steps of an exemplary laser surgical method, according to aspects of this disclosure.

### DETAILED DESCRIPTION

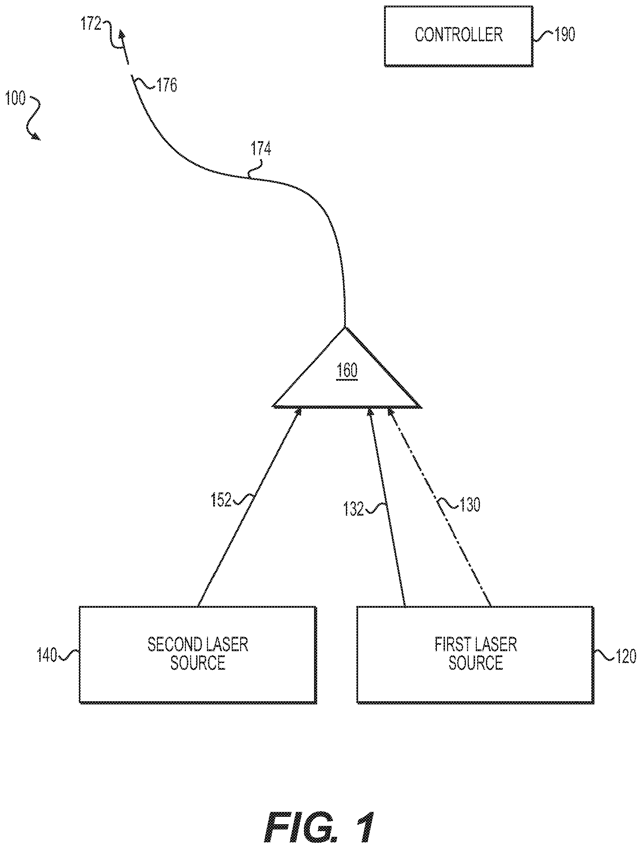

Aspects of the present disclosure are now described more fully hereinafter with reference to FIGs. 1-4. Elements that are identified using the same or similar reference characters refer to the same or similar elements. The various aspects of the present disclosure may, however, be embodied in many different forms and should not be construed as limited to the aspects set forth herein. Rather, these aspects are provided so that this disclosure will be thorough and complete, and will fully convey the scope of any claimed subject matter to those skilled in the art.

Specific details are given in the following description to provide a thorough understanding of aspects of this disclosure. However, it is understood by those of ordinary skill in the art that any aspects described herein may be practiced without these specific details. For example, circuits, systems, networks, processes, frames, supports, connectors, motors, processors, and other components may not be shown, or shown in block diagram form in order to not obscure the described aspects in unnecessary detail.

The terminology used herein is for the purpose of describing particular aspects only and not intended to be limiting of the present disclosure. As used herein, the singular forms "a," "an," and "the" are intended to include the plural forms as well, unless the context clearly indicates otherwise. The terms first, second, etc. are used herein to describe various elements, but these elements should not be limited by these terms. These terms are only used to distinguish one element from another. Thus, a first element could be termed a second element without departing from the teachings of the present disclosure.

As used herein, the terms "comprises" and/or "comprising," or like variation, are intended to cover a non-exclusive inclusion, such that a device or method that comprises a list of elements does not include only those elements, but may include other elements not expressly listed or inherent thereto. For example, the use of these terms to specify the presence of stated features, integers, steps, operations, elements, and/or components does not preclude the presence or addition of one or more other features, integers, steps, operations, elements, components, and/or groups thereof. Conversely, the terms "consists of' and "consisting of' are intended to cover an exclusive inclusion, such that a device or method that consists of a list of elements includes only those elements. Unless stated otherwise, the term "exemplary" is used in the sense of "example" rather than "ideal."

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

It is understood that aspects of the present disclosure may be implemented by computer program instructions. These program instructions may be provided to a processor circuit or controller, such as a microprocessor, microcontroller or other processor, which executes the instructions to implement the functions specified in the block or blocks through a series of operational steps to be performed by the processor(s) and corresponding hardware components.

Aspects of this disclosure are now described with reference to an embodiment of a laser system in accordance with the invention which is configured to output a dual-wavelength laser energy including a first laser energy at a first wavelength and a second laser energy at a second wavelength, wherein the first and second laser energies are output simultaneously, and the first and second wavelengths are different. The laser system is configured to simultaneously output the first laser energy at a wavelength range associated with tissue vaporization, and the second laser energy at a wavelength range associated with tissue coagulation. The vaporization wavelength is in the range of 400nm-600nm, and the coagulation wavelength may is in the range of 780nm-3.0µm. The respective power levels of the first and second laser energies may be modified to achieve a desired laser-tissue interaction. For example, according to one aspect, the thermal denaturation and coagulative effectiveness of the dual-wavelength laser energy may be improved when both of the first and second laser energies are output at low power levels (e.g., 20 and 40 Watts, respectively), or at the same low power lever (e.g., 40W).

According to these aspects, an exemplary laser system 100 in accordance with an embodiment of the invention is depicted in FIG. 1 as being configured to output a dual-wavelength laser energy 172 including a first laser energy 132 and a second laser energy 152. As shown, system 100 includes a first laser source 120 configured to output first laser energy 132, a second laser source 140 configured to output second laser energy 152, a combiner 160 configured to receive first and second laser energies 132 and 152, and output the dual-wavelength laser energy 172. Laser system 100 also may include an optical fiber 174 configured to deliver dual-wavelength laser energy 172 to a targeted location, and a controller 190 configured to operate system 100. Each element of system 100 is now described.

First and second laser sources 120 and 140 may comprise any lasing technologies or laser sources emitting laser energy at any wavelength, power, frequency, etc. An exemplary configuration for laser sources 120 and 140 is described in U.S. Patent Application No. 14/964,890, filed December 10, 2015 (the "'890 Application").

As described in the '890 Application, first laser source 120 is configured to output first laser energy 132 at a first wavelength within a vaporization wavelength range of 400-600nm. In one aspect, the first wavelength may be 532nm. First laser source 120 may be further configured to adjust the power level of first laser energy 132 between a high power level (e.g., 100W) and a low power level (e.g., 20W). As also described in the '890 Application, second laser source 140 is configured to output second laser energy 152 at a second wavelength within a coagulation wavelength range of 780nm-3.0µm. In one aspect, the second wavelength may be 980nm. Second laser source 140 may be likewise configured to adjust the power level of second laser energy 152 between a high power level (e.g., 100W) and a low power level (e.g., 20W).

In FIG. 1, first and second laser sources 120 and 140 are optically coupled to combiner 160. As shown, combiner 160 may be optically coupled to an optical fiber 174 with a distal end 176 configured to output laser energy to the targeted location in either a side-fire or end-fire configuration. Any optical technology may be included in combiner 160, including the optics described in the '890 Application. Combiner 160 may be configured to output the first and second laser energies 132 and 152 through fiber 174 and distal tip 176, individually, sequentially, or simultaneously.

Aspects of system 100 may be operated by controller 190. For example, controller 190 may include one or more processors placed in wired or wireless communication with first and second laser sources 120 and 140. In some aspects, controller 190 may be configured to execute program instructions. Controller 190 is configured to receive input signals from an input device (e.g., control module, foot pedal, triggers, buttons, etc.), and generate a control signal for operating aspects of system 100. For example, system 100 may be switched into different modes and/or selectively activated responsive to one or more control signals generated by controller 190 based on input from the input device.

According to these aspects, laser system 100 of FIG. 1 may be switched into a vaporization mode, in which first laser energy 132 is output from first laser source 120 at a first wavelength of 532nm. The tissue penetration depth of a short wavelength laser, such as 532nm, is typically shorter at higher power levels. Therefore, in the vaporization mode, first laser energy 132 may be output at a high power level of 80W, or 100W, or a greater power level at which first laser energy 132 is configured to vaporize tissue.

Laser system 100 of FIG. 1 may be further switched between the vaporization mode, a single-wavelength coagulation mode, and a dual wavelength coagulation mode. Numerous coagulation modes are described in this application. System 100 may be further configured to switch between any one of these exemplary coagulation modes. The switching between modes may be pre-programmed. In accordance with the present embodiment of the invention, the switching is responsive to user input via, for example, the input devices discussed above.

In the single-wavelength coagulation mode, system 100 may be configured to achieve a first coagulative effect by modifying an aspect of first laser energy 132, outputting first laser energy 132 from combiner 160, and directing energy 132 onto a targeted location from distal end 176 of fiber 174. For example, first laser energy 132 may achieve the first coagulative effect at a first wavelength of 532nm by reducing the power level of first laser energy 132 to 40W. The coagulative effects of first laser energy 132 at this lower power level may be limited by the amount of time required for energy accumulation. For example, because a lengthy irradiation time may be required to accumulate a requisite amount of first laser energy 132 at lower power levels, it may be difficult to provide sufficient tissue coagulation and/or complete hemostasis with only first laser energy 132 in some instances, such as when operating time is limited.

In the dual-wavelength coagulation mode, system 100 may be configured to reduce irradiation time by increasing the speed at which first laser energy 132 accumulates at the targeted location. In this mode, system 100 may be configured to achieve a second coagulative effect by simultaneously outputting first and second laser energies 132 and 152 from combiner 160 as a dual-wavelength laser energy 172. Dual-wavelength laser energy 172 may then be directed onto the targeted location from distal end 176 of fiber 174. First laser energy 132 may be output from first laser source 120 at a first wavelength of 532nm (or other wavelength in the vaporization wavelength range), while second laser energy 152 may be output from second laser source 140 at a second wavelength of 980nm (or other wavelength in the coagulation wavelength range). In some aspects, the power level of first laser energy 132 may be 20W or 40W, while the power level of second laser energy 152 may be 40W.

In this configuration, dual-wavelength laser energy 172 has been proven to realize a second coagulative effect that is considerably greater than the first coagulative effect achievable with first laser energy 132 in the single-wavelength coagulation mode, regardless of treatment speed (e.g., 2, 4, and 6 mm/s). In particular, the second coagulative effect may be realized because the tissue penetration depth of short wavelength lasers has been proven to be greater at lower power levels, such as 20W, where it was previously thought that the coagulative effects of first laser energy 132 would be minimal. Because of this relationship, validated below, second energy 152 may be output simultaneously with first energy 132 as dual-wavelength energy 172 to dramatically increase energy accumulation at the targeted location.

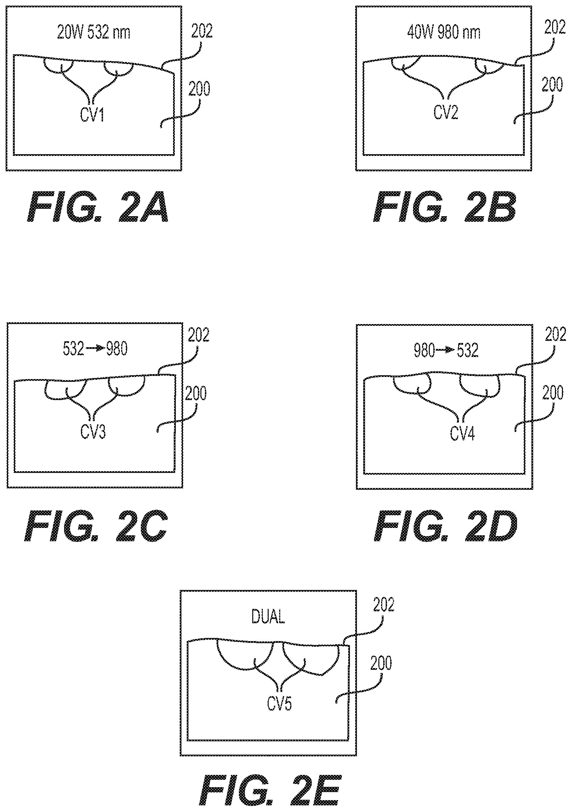

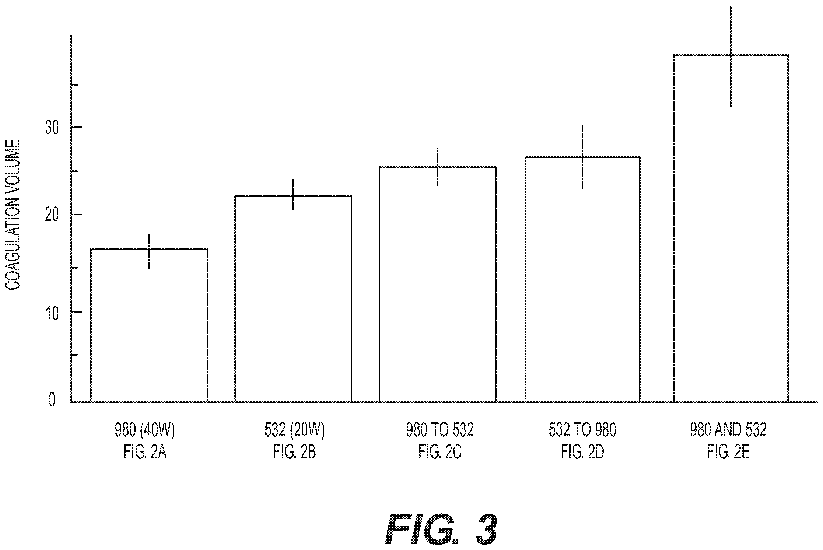

The results described above have been verified in numerous ex vivo trails, wherein the coagulative effects of various laser energies were compared quantitatively in terms of power level, treatment speed, and irradiation mode. During these trials, a coagulation volume was produced with each laser energy. A depiction of the coagulation volumes is shown in FIGs. 2A-2E, and comparison of the volumes is depicted in FIG. 3. It is noted that the coagulation volumes for each test in the trials exhibited minor variations. FIG. 3 depicts the average coagulation volumes for the tests corresponding to each of FIGs. 2A-2E, with the variations illustrated as vertical lines extending above and below the top of the bar graph representing the coagulation volume for each test.

In a first set of trials, kidney tissue 200 having a tissue surface 202 was used as an ex vivo sample. Kidney tissue 200 may be easily procured, and such tests on kidney tissue 200 may be easily reproduced. Within these trials, a number of single-wavelength tests were performed on a targeted location of kidney tissue 200 using either first laser energy 132 at a first wavelength of 532nm and power level of 20W, or second energy at a second wavelength of 980nm and power level of 40W. In one aspect, first laser energy 132 may be Q-switched, and in another aspect, second laser energy 152 may be a continuous wave. A coagulation volume "CV1" produced by first laser energy 132 is depicted in FIG. 2A, and a coagulation volume "CV2" produced by second laser energy 152 is depicted in FIG. 2B. As shown, CV1 is deeper and wider than CV2. In addition, due to properties of kidney tissue 200 (e.g., it being a soft tissue), it was further determined that a working distance of approximately 3mm between tissue surface 202 and fiber tip 174 provided a wide range of power levels where either of the single-wavelength laser energies 132 and 152 may coagulate tissue without vaporization.

Consistent with above, the benefits of single-wavelength coagulation may be realized with system 100 of FIG. 1, for example, by placing system 100 in the single-wavelength coagulation mode and selectively outputting a primary laser energy 130.

As shown in FIGs. 2C and 2D, a number of dual-wavelength tests also were performed on kidney tissue 200 during the first set of trials. For example, two sequential dual-wavelength tests were performed, wherein the targeted location was sequentially irradiated with first and second laser energies 132 and 152. A first set of tests were performed from first laser energy 132 (e.g., at 532nm and 20W) to second laser energy 152 (e.g., at 980nm and 40W), and a second set of tests were performed from second laser energy 152 (e.g., at 980nm and 40W) to first laser energy 132 (e.g., at 532nm and 20W). A coagulation volume "CV3" produced by the first set of tests is depicted in FIG. 2C, and a coagulation volume "CV4" produced by the second set of tests is depicted in FIG. 2D. As shown, CV3 and CV4 are approximately equal. FIGs. 2C-2D also show that sequential dual irradiation produced a coagulation depth similar to that produced with single wavelength irradiation (e.g., FIGs. 2A-2B), but with a coagulation width approximately 15% wider than that produced with single wavelength irradiation. As such, sequential dual irradiation results in a larger coagulation volume, such that CV3 and CV4 are greater than CV1 and CV2.

Also consistent with above, the benefits of sequential dual-wavelength tests may be realized, for example, by outputting first and second laser energies 132 and 152 in a particular sequence. For instance, first laser energy 132 may be delivered, then second laser energy 152 may be delivered, and then first laser energy 132 may be again delivered. First laser energy 132 and second laser energy 152 may be sequentially output for alternating, nonoverlapping periods of time. Alternatively, first laser energy 132 and second laser energy 142 may be sequentially output for alternating, partially overlapping. This alternating application may be repeated as many times as necessary to treat the target tissue.

A number of simultaneous dual-wavelength tests were also performed, wherein the targeted location on kidney tissue 200 was simultaneous irradiated with first and second laser energies 132 and 152. The results of the simultaneous tests are depicted in FIG. 2E, and compared with the results of other laser energy tests in FIG. 3.

As shown in FIG. 2E, simultaneous irradiation with first and second laser energies 132 and 152 produced a coagulation volume "CV5" having a coagulation depth approximately 30% deeper than that of CV1 or CV2 and of CV3 or CV4, and a coagulation width approximately 40% wider than that of CV1 or CV2 and of CV3 or CV4. As such, CV5 is much larger than CV1, CV2, CV3, or CV4. These results were produced consistently regardless of treatment speed, i.e., the speed of fiber movement relative to the targeted location. Moreover, the simultaneous dual-wavelength tests further showed that the coagulation volume, as well as the degree of thermal denaturation, increased approximately linearly with the aggregate power level of first and second laser energies 132 and 152, resulting in a predictable laser-tissue interaction.

The aforementioned benefits of simultaneous dual-wavelength tests may be realized by placing system 100 of FIG. 1 in the dual-wavelength coagulation mode, and outputting a dual-wavelength laser energy 172 including first and second laser energies 132 and 152 at comparable, similar, or identical power levels.

A second set of ex vivo trials were performed to validate the results obtained during the first set of trials. In the second set of trials, porcine skin tissue was used to create a blood-perfused phantom tissue system configured to emulate arterial/venous bleeding. Heparinized rabbit blood was used for validation purposes. Each of the single-wavelength and simultaneous dual-wavelength tests were performed on the porcine tissue using the wavelength and power levels described above with reference to first laser energy 132 and second laser energy 152. These tests confirmed that simultaneous dual-wavelength irradiation was more effective than single-wavelength irradiation. For example, it was determined that both single-wavelength and simultaneous dual-wavelength irradiation could stop bleeding from a small bleeder, such as a 1 mm diameter hole in the tissue. For larger bleeders, however, only dual-wavelength irradiation was effective to reduce or stop bleeding. For example, it was determined that dual-wavelength irradiation could stop bleeding from a 5 mm long cut in the tissue within 6 seconds. Single-wavelength irradiation could not stop or reduce the bleeding from such a cut or wound at all. These results were further confirmed by studying images of the porcine tissue. The images demonstrated that dual-wavelength irradiation produced wider and darker tissue denaturation, as well as deeper and wider coagulation, than otherwise possible with single-wavelength irradiation. Single-wavelength irradiation (e.g., at 532nm) produced superficial ablation at the surface of the tissue and a narrower range of tissue coagulation than the dual-wavelength irradiation. It is noted that the second set of trials also indicated that none of the aforementioned irradiation conditions could stop bleeding when artificial blood (e.g., amaranthine tattoo dye) was used, suggesting that tissue coagulation may require collagen shrinkage as well as thermal blood coagulation to stop bleeding.

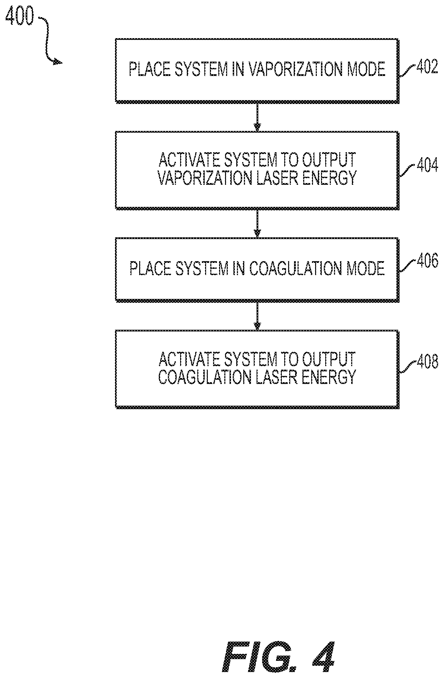

Numerous methods also are described with reference to system 100 of FIGs. 1-3. For example, FIG. 4 shows an exemplary method 400 which does not form part of the invention. In a step 402, a user may place system 100 in the vaporization mode. In a step 404, a user may activate system 100 and output a vaporization laser energy, for example, first laser energy 132 (e.g., at 532nm and 80W). The output vaporization laser energy may be used to perform a cutting, vaporization, ablation, or other laser treatment on a targeted tissue. After beginning the laser treatment, it may be desirable to perform a coagulation operation on the targeted tissue. Accordingly, the user may either switch system 100 from the vaporization mode to any of the coagulation modes described herein. For example, in a step 406, the user may switch system 100 from the vaporization mode to a coagulation mode, for example, the single- or dual-wavelength coagulation mode. In a step 408, the user may activate system 100 and output a coagulation laser energy. The coagulation laser energy may be either second laser energy 152 alone or may be first and second laser energies 132 and 152, as discussed above. The coagulation laser energy may be used to stop bleeding from the treated tissue. The steps of method 400 may be repeated as many times as necessary to effectively treat the tissue.

Any switching of system 100 between the vaporization mode and the first or second coagulation modes may be responsive to an input signal generated by an input device, such as a foot pedal. For example, an exemplary input device is described in U.S. Application No. 12/120,550, filed May 14, 2008. Accordingly, method 400 and other methods disclosed herein may include receiving an input signal with controller 190 and generating a corresponding control signal configured to switch system 100 into the desired mode and/or activate system 100 with the selected laser mode.

While principles of the present disclosure are described herein with reference to illustrative aspects for particular applications, the disclosure is not limited thereto. Those having ordinary skill in the art and access to the teachings provided herein will recognize additional modifications, applications, and aspects all fall in the scope of the aspects described herein. Accordingly, the present disclosure is not to be considered as limited by the foregoing description.

## Claims

1. A laser system (100) comprising:
a first laser source (120) configured to output a first laser energy at a first wavelength;
a second laser source (140) configured to output a second laser energy at a second wavelength;
a combiner (160) configured to receive the first and second laser energies and output a dual-wavelength laser energy, wherein the first and second wavelengths are different, and to output said first and second laser energies simultaneously, wherein the first wavelength occurs within a vaporization wavelength range of 400nm-600nm, and the second wavelength occurs within a coagulation wavelength range of 780nm-3.0µm; and
a controller (190) configured to receive user input from an input device, wherein the controller (190) is configured to place the laser system (100) in a vaporization mode to output the first laser energy onto a targeted location, and wherein the controller (190) is configured to place the laser system (100) in a dual-wavelength coagulation mode to output the dual-wavelength laser energy onto the targeted location, responsive to said user input.

2. The laser system (100) of claim 1, wherein the first wavelength is 532nm, and the second wavelength is 980nm.

3. The laser system (100) of claim 1, wherein the first laser energy is output from the first laser source (120) at a first power level, and the second laser energy is output from the second laser source (140) at a second power level.

4. The laser system (100) of claim 3, wherein the first power level is 20W, and the second power level is 40W.

5. The laser system (100) of claim 3, wherein the first power level is identical to the second power level.

6. The laser system (100) of claim 1, wherein the first laser energy is output as a pulsed waveform, and the second laser energy is output as a continuous waveform.

7. The laser system (100) of claim 1, wherein the combiner (160) is optically coupled to an optical fiber (174) having a distal end (176).

8. The laser system (100) of claim 7, wherein the distal end (176) includes a side-fire configuration.

9. The laser system (100) of claim 7, wherein the distal end (176) includes an end-fire configuration.

10. The laser system (100) of claim 1, wherein the input device is a control module, a foot pedal, one or more triggers, or one or more buttons.

11. The laser system of claim 10, wherein the laser system (100) is switchable between at least the vaporization mode and the dual-wavelength coagulation mode and selectively activatable based on input from the input device.

12. The laser system of claim 11, wherein the laser system (100) is switchable between at least the vaporization mode, the dual-wavelength coagulation mode and a single-wavelength coagulation mode.

## Patentansprüche

1. Lasersystem (100), umfassend:
eine erste Laserquelle (120), dazu ausgelegt, eine erste Laserenergie in einer ersten Wellenlänge auszugeben;
eine zweite Laserquelle (140), dazu ausgelegt, eine zweite Laserenergie in einer zweiten Wellenlänge auszugeben;
einen Kombinierer (160), dazu ausgelegt, die erste und zweite Laserenergie zu empfangen und Laserenergie in zwei Wellenlängen auszugeben, wobei die erste und zweite Wellenlänge unterschiedlich sind, und die erste und zweite Laserenergie gleichzeitig auszugeben, wobei die erste Wellenlänge innerhalb eines Verdampfungswellenlängenbereichs von 400 nm-600 nm auftritt und die zweite Wellenlänge innerhalb eines Koagulationswellenlängenbereichs von 780 nm-3,0 µm auftritt; und
eine Steuerung (190), dazu ausgelegt, Benutzereingabe von einer Eingabevorrichtung zu erhalten, wobei die Steuerung (190) dazu ausgelegt ist, das Lasersystem (100) in einen Verdampfungsmodus zu versetzen, um die erste Laserenergie auf einen angestrebten Ort auszugeben, und wobei die Steuerung (190) dazu ausgelegt ist, das Lasersystem (100) in einen Koagulationsmodus mit zwei Wellenlängen zu versetzen, um die Laserenergie in zwei Wellenlängen auf den angestrebten Ort als Reaktion auf die Benutzereingabe auszugeben.

2. Lasersystem (100) nach Anspruch 1, wobei die erste Wellenlänge 532 nm ist und die zweite Wellenlänge 980 nm ist.

3. Lasersystem (100) nach Anspruch 1, wobei die erste Laserenergie in einem ersten Energieniveau von der ersten Laserquelle (120) ausgegeben wird und die zweite Laserenergie in einem zweiten Energieniveau von der zweiten Laserquelle (140) ausgegeben wird.

4. Lasersystem (100) nach Anspruch 3, wobei das erste Energieniveau 20 W ist und das zweite Energieniveau 40 W ist.

5. Lasersystem (100) nach Anspruch 3, wobei das erste Energieniveau identisch mit dem zweiten Energieniveau ist.

6. Lasersystem (100) nach Anspruch 1, wobei die erste Laserenergie als eine gepulste Wellenform ausgegeben wird und die zweite Laserenergie als eine kontinuierliche Wellenform ausgegeben wird.

7. Lasersystem (100) nach Anspruch 1, wobei der Kombinierer (160) optisch an eine optische Faser (174) mit einem distalen Ende (176) gekoppelt ist.

8. Lasersystem (100) nach Anspruch 7, wobei das distale Ende (176) eine Seiten-Zündkonfiguration aufweist.

9. Lasersystem (100) nach Anspruch 7, wobei das distale Ende (176) eine End-Zündkonfiguration aufweist.

10. Lasersystem (100) nach Anspruch 1, wobei die Eingabevorrichtung ein Steuerungsmodul, ein Fußpedal, ein oder mehrere Trigger oder eine oder mehrere Tasten ist.

11. Lasersystem nach Anspruch 10, wobei das Lasersystem (100) zwischen mindestens dem Verdampfungsmodus und dem Koagulationsmodus mit zwei Wellenlängen umschaltbar und basierend auf Eingabe von der Eingabevorrichtung wahlweise aktivierbar ist.

12. Lasersystem nach Anspruch 11, wobei das Lasersystem (100) zwischen mindestens dem Verdampfungsmodus, dem Koagulationsmodus mit zwei Wellenlängen und einem Koagulationsmodus mit einer einzelnen Wellenlänge umschaltbar ist.

## Revendications

1. Système de laser (100) comprenant :
une première source de laser (120) qui est configurée pour émettre en sortie une première énergie de laser à une première longueur d'onde ;
une seconde source de laser (140) qui est configurée pour émettre en sortie une seconde énergie de laser à une seconde longueur d'onde ;
un combineur (160) qui est configuré pour recevoir les première et seconde énergies de laser et pour émettre en sortie une énergie de laser à deux longueurs d'onde, dans lequel les première et seconde longueurs d'onde sont différentes, et pour émettre en sortie lesdites première et seconde énergies de laser de façon simultanée, dans lequel la première longueur d'onde se trouve à l'intérieur d'une plage de longueurs d'onde de vaporisation qui va de 400 nm à 600 nm, et la seconde longueur d'onde se trouve à l'intérieur d'une plage de longueurs d'onde de coagulation qui va de 780 nm à 3,0 µm ; et
un contrôleur (190) qui est configuré pour recevoir une entrée d'utilisateur en provenance d'un dispositif d'entrée, dans lequel le contrôleur (190) est configuré pour placer le système de laser (100) dans un mode de vaporisation pour qu'il émette en sortie la première énergie de laser sur une localisation ciblée, et dans lequel le contrôleur (190) est configuré pour placer le système de laser (100) dans un mode de coagulation à deux longueurs d'onde pour qu'il émette en sortie l'énergie de laser à deux longueurs d'onde sur la localisation ciblée, en réponse à ladite entrée d'utilisateur.

2. Système de laser (100) selon la revendication 1, dans lequel la première longueur d'onde est de 532 nm, et la seconde longueur d'onde est de 980 nm.

3. Système de laser (100) selon la revendication 1, dans lequel la première énergie de laser est émise en sortie depuis la première source de laser (120) à un premier niveau de puissance, et la seconde énergie de laser est émise en sortie depuis la seconde source de laser (140) à un second niveau de puissance.

4. Système de laser (100) selon la revendication 3, dans lequel le premier niveau de puissance est de 20 W, et le second niveau de puissance est de 40 W.

5. Système de laser (100) selon la revendication 3, dans lequel le premier niveau de puissance est identique au second niveau de puissance.

6. Système de laser (100) selon la revendication 1, dans lequel la première énergie de laser est émise en sortie en tant que forme d'onde pulsée, et la seconde énergie de laser est émise en sortie en tant que forme d'onde continue.

7. Système de laser (100) selon la revendication 1, dans lequel le combineur (160) est couplé optiquement à une fibre optique (174) qui comporte une extrémité distale (176).

8. Système de laser (100) selon la revendication 7, dans lequel l'extrémité distale (176) inclut une configuration à tir latéral.

9. Système de laser (100) selon la revendication 7, dans lequel l'extrémité distale (176) inclut une configuration à tir d'extrémité.

10. Système de laser (100) selon la revendication 1, dans lequel le dispositif d'entrée est un module de commande, un levier de commande à pied, un ou plusieurs moyen(s) de déclenchement, ou un ou plusieurs bouton(s).

11. Système de laser selon la revendication 10, dans lequel le système de laser (100) peut être commuté entre au moins le mode de vaporisation et le mode de coagulation à deux longueurs d'onde et peut être activé de façon sélective sur la base d'une entrée en provenance du dispositif d'entrée.

12. Système de laser selon la revendication 11, dans lequel le système de laser (100) peut être commuté entre au moins le mode de vaporisation, le mode de coagulation à deux longueurs d'onde et un mode de coagulation à une seule longueur d'onde.
